# EUROPEAN PATENT APPLICATION

(11) **EP 4 832 010 A1**
(43) Date of publication of application: **28.10.2026**
(21) Application number: 25172732.7
(22) Date of filing: 25.04.2025
(51) Int. Cl.: G16H 50/30, G16H 50/70, A61B 5/01, A61B 5/024, A61B 5/08, A61B 5/11, A61B 5/00

(54) **METHOD AND SYSTEM FOR PREDICTING A DISEASE STATUS INDICATOR AND PROGRESSION THEREOF FOR PATIENTS SUFFERING FROM COPD**

(71) Applicant: Merck Patent GmbH, 64293 Darmstadt (DE)
(72) Inventor: SCHNEIDER, Armin, 69198 Schriesheim (DE); VIVAS PRIETO, Sonia Victoria, 76698 UBSTADT-WEIHER (DE); ION, Catalin, 63225 Langen (DE); SPOELGEN, Robert, 61476 Kronberg (DE)
(74) Representative: Merck Patent Association

(57) **Abstract**

The invention provides a computer-implemented (inference) method for providing a CAT-score prediction (43) for a target subject (55) and a target date using a trained model (33), wherein the computer-implemented method comprises: generating data features (15) from physiological data (10) on the target subject (55), wherein the physiological data (10) comprises electrocardiogram data (11), temperature data (12), and motion data (13), and wherein the data features (15) comprise a respiration rate feature, a heart rate variability feature, a heart rate feature, and an activity feature; and providing input data (5) to the trained model (33) and generating, by the trained model (33), the CAT-score prediction (43) based on the input data (5), wherein the input data (5) comprises (i) the data features (15), (ii) prior CAT-scores (40), and (iii) target subject information (20) for the target subject (55), and target subject information (20), wherein the prior CAT-scores (40) precede the target date, wherein the target subject information (20) comprises a subject identifier for the target subject (55).

## Description

### FIELD OF THE INVENTION

The invention relates to a computer-implemented method for predicting a disease status indicator and progression thereof for patients, for example by predicting a CAT-score for patients suffering from COPD. The invention further relates to a computer-implemented method for providing a trained model, to the trained model, and to a data carrier comprising the trained model. The invention further relates to a data processing system and to a system comprising the data processing system. The invention further relates to a computer program product and a data carrier comprising instructions for executing the computer implemented method(s).

### BACKGROUND OF THE INVENTION

Methods for predicting CAT-scores are known in the art. For instance, Xiao et al., Breathing New Life into COPD Assessment: Multisensory Home-monitoring for Predicting Severity, INTERNATIONAL CONFERENCE ON MULTIMODAL INTERACTION (ICMI '23), October 09-13, 2023 describe a data pre-processing and feature engineering pipeline for multimodal data from remote home-monitoring of COPD patients. The article describes models for forecasting the absolute and differenced COPD Assessment Test (CAT) scores based on multisensory data.

### SUMMARY OF THE INVENTION

Chronic Obstructive Pulmonary Disease (COPD) is a progressive lifethreatening lung disease for which currently no curative treatment is available. COPD may be the fourth most frequent cause of death worldwide and is associated with high costs for treating and managing patients. As described in the 2024 report of the Global Initiative for Chronic Obstructive Lung Disease (GOLD): "COPD is a heterogeneous lung condition characterized by chronic respiratory symptoms (dyspnea, cough, sputum production, exacerbations) due to abnormalities of the airways (bronchitis, bronchiolitis) and/or alveoli (emphysema) that cause persistent, often progressive airflow obstruction."

COPD may be characterized by fluctuations in symptom severity over time, the most well-defined of which are moderate and severe exacerbations. These fluctuations may have a strong impact on quality of life and on disease progression. In part due to this fluctuating nature of the disease, physicians typically only have momentary glimpses into the disease status every 3 or 6 months rather than a view of disease progression over time.

The COPD Assessment Test (CAT) is a short digital or paper questionnaire for a patient to provide a proxy for the disease status in this patient. The CAT is used in routine clinical practice and in clinical interventional and observational studies, including randomized controlled trials, as a proxy of health-related quality of life (HRQoL) and clinical efficacy. The CAT results in a CAT-score between 0 and 40, wherein higher scores indicate more severe impact of COPD on the patient's life as reported by the patient. Although the CAT-score relies on subjective answers from a patient, the CAT-score may generally be considered as a useful indicator for a physician regarding the disease status and disease progression in the patient.

However, physician visits may be relatively infrequent, and at-home questionnaires may typically have a low response rate for at least part of the patient population. Further, it may be a substantial burden on a patient to regularly fill in the CAT questionnaire. Hence, in practice, even with the CAT, physicians may only have access to occasional snapshots of the (subjective) patient-reported disease status.

The prior art may describe the use of accelerometer parameters obtained using a wearable device together with a home monitoring system that measures cough, sputum, spirometry values, and air quality, to predict the next day CAT-score from data up to 3 days in the past. Such an approach may facilitate obtaining regular CAT-scores without directly relying on patient adherence to filling out CAT questionnaires. The prior art may further describe such approaches for predicting a next-day CAT-score. However, the required home monitoring system may be relatively complex, while the overall error for absolute CAT-score predictions may be relatively high, which may preclude clinical usage.

Further, some prior art approaches may require a subject to perform specific activities, such as physical exercises, in order to make a prediction, which may be prohibitive for daily use within the broader COPD patient population.

Hence, it is an aspect of the invention to provide an alternative method for predicting a CAT-score for a subject, which method preferably further at least partly obviates one or more of above-described drawbacks. The present invention may have as object to overcome or ameliorate at least one of the disadvantages of the prior art, or to provide a useful alternative.

Accordingly, the invention may provide a method, especially a computer-implemented (inference) method, for providing a COPD Assessment Test score prediction (or "CAT-score prediction"), specifically for a target subject and a target date. The computer-implemented method may involve the use of a (pre-)trained (machine learning) model. The computer-implemented method may comprise a data processing stage and an inference stage. The method may comprise, in the data processing stage, generating (or "extracting") data features from physiological data on (or "from") the target subject, especially wherein the physiological data comprises electrocardiogram data, temperature data, and motion data (on the target subject), such as motion data from an accelerometer and/or a gyroscope. In embodiments, the data features may comprise (at least) a respiration rate feature, a heart rate variability feature, a heart rate feature, and an activity feature. The method may further comprise, in the inference stage, providing input data to the trained model and generating, by the trained model, the CAT-score prediction based on the input data. The trained model may thus be configured to generate the CAT-score prediction based on the input data. The input data may especially comprise the data features, prior CAT-scores for the target subject, and target subject information. The prior CAT-scores may comprise user-reported prior CAT-scores and/or predicted prior CAT-scores. Further, the prior CAT-scores may precede the target date, i.e., the prior CAT-scores may correspond to timepoints prior to (e.g., days before) the target date. The target subject information may especially comprise a subject identifier (code) for the target subject.

In specific embodiments, the invention provides a computer-implemented method for providing a CAT-score prediction for a target subject and a target date using a trained model, wherein the computer-implemented method comprises: generating data features from physiological data on the target subject, wherein the physiological data comprises electrocardiogram data, temperature data, and motion data, and wherein the data features comprise a respiration rate feature, a heart rate variability feature, a heart rate feature, and an activity feature; providing input data to the trained model and generating, by the trained model, the CAT-score prediction based on the input data, wherein the input data comprises the data features, prior CAT-scores for the target subject, and target subject information, wherein the prior CAT-scores precede the target date, wherein the target subject information comprises a subject identifier for the target subject.

The invention may facilitate providing accurate CAT-score predictions days in advance, while using physiological data obtainable using relatively common and portable sensor modalities, i.e., without requiring complex dedicated home monitoring systems. **In** particular, machine learning models have been generated that can accurately predict CAT-scores multiple days, e.g. 6 days, in advance (see experiments below) using input data including data features generated based on the physiological data. Surprisingly, the inventors realized that the model performance substantially benefits from a subject identifier representing the subject identity as well as from prior CAT-scores for the subject (see further below). Hence, the computer-implemented method may relate to providing a CAT-score prediction using a trained model and input data comprising (a) data features generated from the physiological data, (b) a subject identifier (code), and (c) prior CAT-scores. In particular, the prior CAT-scores may be obtained using a separate (second) trained model configured to predict a concurrent CAT-score for a given day from same-day physiological data (see below).

The invention may thus provide a method, especially a computer-implemented method, for providing a CAT-score prediction for a target subject and a target date using a trained model. As such, the invention provides a method for predicting a disease status indicator and progression thereof for patients suffering from COPD.

The term "computer-implemented method" herein refers to a method that is carried out by a computer system (or "data processing system"), particularly wherein the computer system carries out one or more steps of the method, such as all steps of the method. The computer-implemented method may, for brevity, hereinafter also be referred to as "the method" or "the prediction method".

The term "CAT-score prediction" herein refers to a CAT-score that is predicted rather than (directly) derived from a CAT questionnaire. In particular, the CAT-score prediction may be for a different day than for which any user-reported CAT-scores (from a CAT questionnaire) for the subject are available.

The term "target subject" may herein refer to a specific individual, particularly a COPD patient, for which a CAT-score prediction is made. The target subject may especially be a human target subject.

The phrase "providing a CAT-score prediction for a target subject and a target date" and similar phrases herein refer to the CAT-score prediction specifically corresponding to a prediction of the target subject's CAT-score on the target date.

The term "trained model" herein refers to a machine learning or artificial intelligence model that has undergone a training process (see further below), during which the model has learned to make predictions, classifications, or decisions based on input data comprising specific data categories. The trained model may especially be configured for inference, i.e., for the making of predictions based on new input data (not used during model training). The trained model may especially be a machine learning model.

The method may comprise one or more of a data collection stage, a data processing stage, a loading stage, an inference stage, a post-processing stage, and a transmission stage, especially at least the data processing stage and the inference stage.

In embodiments, the method may comprise the data collection stage. In such embodiments, the method, especially the data collection stage, may comprise acquiring, such as receiving, the physiological data (on the subject), such as from a sensor arrangement. The sensor arrangement may especially comprise an electrocardiogram sensor, a temperature sensor, and a motion sensor, such as an accelerometer and/or a gyroscope. The physiological data may thus especially comprise (at least) one or more of electrocardiogram data (or "ECG data"), temperature data, and motion data (on the target subject). In specific embodiments, the sensor arrangement may be integrated in a wearable device, wherein the sensor arrangement is configured to gather physiological data from the wearer of the wearable device. In embodiments, the method may, for instance, comprise accessing an (online) data repository for accessing the physiological data (on the subject). In further embodiments, the method may comprise receiving the physiological data, e.g., from a sensor arrangement configured to continuously or intermittently, e.g., regularly, submit physiological data to a data processing system executing the method.

The method may further comprise the data processing stage. The method, especially the data processing stage, may comprise generating data features from the physiological data (on the target subject), especially wherein the physiological data comprises one or more of electrocardiogram data, temperature data, and motion data. Especially, in embodiments, the physiological data comprises (at least) the electrocardiogram data, the temperature data, and the motion data.

As will be known to the person skilled in the art of machine learning models, feature generation (or "feature engineering") may be an important step in machine learning, as this step may involve the transformation of raw data into data features suitable and meaningful for model training (see below) and for inference. In particular, the data processing stage may comprise (a) preprocessing of the physiological data, e.g., comprising one or more of handling missing values, correcting errors, filtering out noise, standardizing (or normalizing), data type conversions (e.g., categorical to numerical), and numerical scaling, (b) generating of the features based on the (pre-processed) physiological data, and (c) converting the data features into a suitable format for the trained model (if needed).

A large variety of potential data features may be extracted from the physiological data, specifically from the electrocardiogram data, temperature data, and motion data. In an extensive evaluation of different potential data features (see below), the following data features were determined to be particularly relevant for accurate CAT-score predictions: a respiration rate feature, a heart rate variability feature, a heart rate feature, and an activity feature. Hence, in embodiments, the data features comprise (at least) a respiration rate feature, a heart rate variability feature, a heart rate feature, and an activity feature. In particular, the inclusion of these data features was identified to be particularly beneficial for model performance.

The term "data feature" may herein refer to any feature derived from the (raw) physiological data and usable for model training and/or inference. In general, the data features may result from physiological data pre-processing and subsequent feature generation.

The term "a parameter feature" and similar terms, such as "a respiration rate feature" and "a heart rate variability feature", refers to a feature related to the indicated parameter. For instance, the term "a respiration rate feature" may relate to a respiration rate, such as a daily respiration rate, an average hourly respiration rate (on a given day), or a median hourly respiration rate (on a given day), and/or to a breathing episode, such as to an atypical breathing episode, optionally including one or more of a breathing rate during the breathing episode and an episode duration of the breathing episode. It will be clear to the person skilled in the art that various (related) features regarding the "respiration rate" of a subject may be generated. For instance, the respiration rate feature may comprise a median (daily) respiration rate, a (daily) respiration rate variance, a (daily) respiration rate standard deviation, a minimum (daily) respiration rate, a maximum (daily) respiration rate, a (mean/median) (daily) inspiration/expiration ratio, or variance or standard deviation thereof, and a respiration rate relative to an activity feature. For explanatory purposes, the features may herein primarily be described in the context of "daily" (summary) features. However, alternatives such as "daytime" or "nighttime" features may also be used.

For instance, in embodiments, the respiration rate feature may be selected from the group comprising an atypical breathing episode feature, especially including a breathing rate and episode duration of the atypical breathing episode, and a (daily) respiration rate, such as a mean respiration rate, or such as a median respiration rate.

In embodiments, the respiration rate may be derived from (pre-processed) physiological data, particularly from (pre-processed) electrocardiogram data, using a peak finding algorithm (see below).

As described above, the data features may further comprise a heart rate variability feature. In embodiments, the heart rate variability feature may be selected from the group comprising a standard deviation of an interbeat interval, especially SDNN (standard deviation of Normal-to-Normal intervals) or SDRR (standard deviation of RR intervals), a stress index (SI), and a proportion of successive Normal-to-Normal intervals exceeding a predefined duration, e.g., 50 ms for pNN50.

In embodiments, the heart rate variability rate may be derived from (pre-processed) physiological data, particularly from (pre-processed) electrocardiogram data, using a peak finding algorithm (see below). Similarly, a peak finding algorithm may be used to determine the heart rate feature.

In particular, in embodiments, the data features further comprise a heart rate feature. The heart rate feature may be selected from the group comprising a mean (daily) heart rate, a maximum heart rate (on a day), and a tachycardia episode feature, optionally covering a number of occurrences and their duration. The term "tachycardia episode feature" may herein especially correspond to a time period during which the (estimated) heart rate of the subject is at least 100 bpm.

It will be clear to the person skilled in the art that the term "heart rate feature" relates to the heart rate as such, whereas the term "heart rate variability feature" specifically relates to the variability in the heart rate.

The data features may further comprise an activity feature. The term "activity feature" may herein refer to any feature characterizing how active the subject is (or "was"). The activity feature may, for instance, relate to a sleep feature. Alternatively, the activity feature may distinguish periods of non-activity (e.g., sitting, lying, standing), low activity (walking and fast-walking), and high activity (walking up and down stairs, running, exercise). The activity feature may further relate to a step count. The activity feature may further relate to an activity frequency, e.g., the frequency of activity above a pre-defined threshold. In further embodiments, the activity feature may especially comprise a movement feature, i.e., an activity feature related to movement. The activity feature may further relate to one or more motion data clusters, such as clusters defined using a K-means clustering algorithm on accelerometer data (of a plurality of subjects; see below). In specific embodiments, the activity feature may be selected from the group comprising a motion data cluster (based on K-means clustering), an activity frequency, and a step count.

Besides above-mentioned data features, also other data features appear beneficial for model performance. For instance, model performance may also benefit from the inclusion of one or more of a temperature feature and a cough feature.

Hence, in embodiments, the data features further comprise a temperature feature. The term "temperature feature" may herein specifically refer to a body temperature feature. The temperature feature may especially be selected from the group comprising a minimum (daily) temperature and a maximum (daily) temperature. The temperature feature may especially be derived (at least) from the temperature data.

In further embodiments, the data features may comprise a cough feature. The cough feature may be selected from the group comprising a cough frequency and a cough frequency change. The cough feature may especially be generated based on one or more of the electrocardiogram data and the motion data. In specific embodiments, the cough feature may be generated based on the electrocardiogram data and the motion data using a gradient boosting regressor, wherein the gradient boosting regressor was (pre-)trained to predict the cough feature based on electrocardiogram and motion data.

The data features may, in embodiments, further comprise trend features (also see further below). In particular, the trend features may indicate the trend in a particular parameter, such as a temperature trend over the course of multiple days. In embodiments, the trend features may comprise a lag transformation, such as a moving average, rolling statistic, expanding window statistic and/or difference. In further embodiments, the trend features may include a non-transformed data feature at a lag time, e.g., a data feature from yesterday may be considered for the present day.

Data features from several prior days (including the present day) may be used for the models, for example for a total of 14, 10, 8, up to one day in the past. It will be clear to the person skilled in the art that the use of trend features may depend on the used model and the training algorithm. For example, boosting algorithms like XGBoost or LightGBM work well with trend data, whereas for more explicit time series models like LSTM or 1D CNN or mixed LSTM / 1D CNN untransformed primary data features from n days in the past may be primarily used.

In embodiments, the method may further comprise the loading stage. In such embodiments, the method, especially the loading stage, may comprise loading the (pre-)trained model, such as loading the model into (working) memory. In particular, the loading stage may comprise loading a trained model previously trained using training data to provide a CAT-score prediction.

In further embodiments, the method may comprise the inference stage. The method, especially the inference stage, may comprise providing input data to the trained model and generating, by the trained model, the CAT-score prediction based on the input data. Accordingly, the trained model may be configured to provide the CAT-score prediction based on the input data (or "the trained model may be configured to infer the CAT-score prediction from the input data").

The input data may comprise the data features. In particular, the input data may comprise the data features generated during the processing stage (see above).

The input data may further comprise target subject information. The target subject information may comprise a subject identifier (code). In particular, as the CAT-score is a subjective score, the subjective patient experience on symptoms will vary between patients, and the subject identifier - representing patient identity - may serve to capture an individual translation factor. Surprisingly, the subject identifier ranked as one of the top features in model performance (see below).

In further embodiments, the subject information may comprise baseline features and/or demographic data. For instance, the baseline features may comprise a weight and/or a value relating to an initial exhalation volume, such as the initial amount of air exhaled during the first second of a forced exhale, i.e., the initial Forced Expiratory Volume for 1 second (FEV1-value). Other baseline features (covariates) may be: GOLD stage (for COPD patients), history of exacerbations (for COPD patients), oscillometry parameters at baseline (e.g., Resistance at 5 Hz (R5), Difference between Resistance at 5 Hz and Resistance at 20 Hz (R5-20), Difference between Resistance at 5 Hz and Resistance at 19 Hz (R5-19), Area under a reactance curve (AX), Reactance at 5 Hz (X5)), Spirometry/ plethysmography at baseline (e.g., Forced Expiratory Volume in 1 second (FEV1), Forced Vital Capacity (FVC), Ratio of Forced Expiratory Volume in 1 second to Forced Vital Capacity (FEV1/FVC), Forced Expiratory Flow between 25% and 75% of FVC (FEF 25-75), Maximum Voluntary Ventilation (MVV), Vital Capacity (VC) ,Residual Volume (RV), Total Lung Capacity (TLC), Functional Residual Capacity (FRC)), diffusion coefficients (e.g., Diffusing capacity for carbon monoxide (DLCO)), blood gas parameters (e.g., oxygen saturation, partial pressures of oxygen and/or carbon dioxide, acidity (pH) or base excess (BE)) and eosinophil counts, neutrophil counts, CRP (C-reactive protein). The demographic data may comprise one or more of age, gender, sex, ethnicity, nationality, residence location, and culture group. In particular, different social groups may have and/or report different (subjective) experiences on symptoms, such as different pain experiences, which may lead to different (subjective) CAT-scores for objectively similar disease states.

The trained model may thus also be trained (in part) on data pertaining to the target subject. In particular, the model may be trained using data from a subject population including the target subject (see the computer-implemented training method below). In embodiments, the trained model is trained (or 'was trained') to provide CAT-score predictions using training data. The training data may - for (each subject in) a subject population - include training data features, training prior CAT-scores, especially one or more of training past model predictions and training subject-reported CAT-scores, subject information, and target CAT-scores. In particular, the subject population may include the target subject (with corresponding subject information). In embodiments, the training data may cover a target subject training timeframe (for the target subject) of at least 14 days, such as at least 30 days, especially at least 60 days. In further embodiments, for at least part of the subject population, the training data may cover a subject training timeframe of at least 30 days, such as at least 45 days, especially at least 60 days. As will be clear to the person skilled in the art, the training data may especially comprise the same data types as the input data, i.e., the trained model may be trained using the same data types as that will later be provided to the model for inference. In particular, in embodiments, the training data features and the data features include the same feature types. Especially, the training data features and the data features consist of the same feature types.

The trained model may thus be trained using a subject population including the target subject. By including data from a larger subject population, the machine learning model may identify general relationships between the data features and the CAT-score, which may benefit from different subjects having different experiences on symptoms during the training timeframe. Training using a subject population may lead to a substantially better performing model than if training would be performed based solely on the target subject. However, due to individual differences between subjects, and possibly due to the CAT-score being subjective, the inclusion of training data on the specific target subject substantially improves the CAT-score predictions for the target subject.

The phrase "the data covers a timeframe" and similar phrases used herein refer to the data including values (or "measurements") for a plurality of timepoints (or "observations") across the timeframe. For instance, the data may cover daily values within the timeframe, especially for the data features and for the prior CAT-scores. It will be clear to the person skilled in the art that (at least part of) the subject information, such as the subject identifier, will typically be constant (within the timeframe).

Accordingly, the input data may comprise a plurality of timepoints, wherein for each timepoint the input data includes (optionally imputed) values for the data features and for the prior CAT-scores. Similarly, in embodiments, the training data may comprise a plurality of training timepoints, wherein for each training timepoint the training data includes (optionally imputed) values for the training data features and for the training prior CAT-scores.

In embodiments, the data features and the prior CAT-scores correspond to a timeframe of n days, wherein n ≥ 1, especially n ≥ 3, such as n ≥ 5. Accordingly, in embodiments, the physiological data may correspond to the timeframe of n days. In particular, the data features, the prior CAT-scores and the physiological data may correspond to the same timeframe. In general, the n days may be consecutive. However, the timeframe may also contain gaps, e.g., wherein data is available for weekdays but not for the weekend, or wherein data is missing for a single day. In such embodiments, the input data may cover at least 50% of days in the timeframe, such as at least 60%, especially at least 75%, such as at least 90%. In embodiments, the missing data may be imputed, e.g. for time series models or for calculating trend features.

For instance, in embodiments, the inference method may comprise imputing missing features using one or more of a Last Observation Carried Forward (LOCF), a mean value, a median value, a mode, a k-nearest neighbor value, a Multivariate Imputation by Chained Equations (MICE), or a recursive imputation approach.

In embodiments, the target data for the CAT-score prediction may be the present date. In particular, in embodiments, the timeframe (for the data features and the prior CAT-scores) may include a last date (or "last input date"), and the target date may be the last date. The term "last date" may herein refer to the most recent date within the considered timeframe.

As described above, the target data for the CAT-score prediction may especially be a future date. In particular, the timeframe (for the data features and the prior CAT-scores) may include a last date (or "last input date"), and the target date may be selected from the range of 1 - 14 days after the last date, such as 2-10 days after the last date, especially 5-7 days after the last date.

The term "target date" may also refer to a plurality of target dates. In particular, in embodiments, the target date comprises a plurality of target dates selected from the range of 1 - 14 days after the last date, such as from the range of 2 - 10 days after the last date, especially from the range of 5 - 7 days after the last date. In such embodiments, the method, especially the inference stage, may comprise generating a CAT-score prediction for each of the plurality of target dates.

As mentioned above, the input data may further comprise prior CAT-scores. The CAT-score may typically be highly autocorrelated over time within a subject. Accordingly, the prior CAT-scores were found to be among the top features for the CAT-score prediction. Surprisingly, however, even CAT-scores of several days prior to a target date, without the intermediate days being covered, such as CAT-scores for 6-10 days before the target date, were among the top performing features for the CAT-score prediction. Further, during modeltraining user-reported prior CAT-scores may be used, but such prior CAT-scores may be less readily available after model training. The inventors surprisingly realized that subject-reported CAT-scores may be complemented by - or even replaced by - concurrent CAT-score predictions for days for which the physiological data is available. In particular, a second trained (machine learning) model may predict a concurrent CAT-score for days for which physiological data is available, thus limited in time to the present day or days prior thereto, to provide predicted prior CAT-scores. In such embodiments, the user input (for the (first) trained model) may comprise the predicted prior CAT-scores.

Hence, in embodiments, the computer-implemented method may comprise a second data processing stage (or "prior data processing stage") and a second inference stage (or "prior inference stage"). The method, especially the second data processing stage, may comprise generating second data features from the physiological data, especially wherein the second data features comprise a respiration rate feature, a heart rate variability feature, a heart rate feature, and an activity feature. Further, the method, especially the second inference stage, may comprise providing second input data to a second trained model, and generating, by the second trained model, at least part of the prior CAT-scores. Accordingly, the second trained model may (be configured to) provide second CAT-score predictions based on the second input data. The second input data may comprise the second data features and the target subject information. In particular, the second model may (be configured to) provide second CAT-score predictions for timepoints (or "days") corresponding to the timepoints of the second data features (or "of the physiological data").

The data features and the second data features may comprise the same data feature types, i.e., the data features and the second data features may both contain a respiration rate feature, a heart rate variability feature, a heart rate feature, and an activity feature. In embodiments, the data features and the second data features may be the same, i.e., in embodiments, the method may comprise a single data processing stage to generate the data features, wherein the input data and the second input data both comprise the (same) data features. However, the data features and the second data features may also differ. For instance, the data features may comprise a mean (daily) heart rate as heart rate feature, whereas the second data features may comprise a median (daily) heart rate (or *vice versa).* In particular, for both the trained model and the second trained model, the data feature types may be selected in view of prediction performance.

In embodiments, the second trained model may be configured to provide a second CAT-score prediction for a second target date, based on data features (solely) for the second target date, i.e., the second trained model may (be configured to) operate on data from a single day (at a time).

In further embodiments, the second trained model is trained (or 'was trained') to provide second CAT-score predictions using second training data, wherein the second training data includes - for (each subject in) a subject population with - second training data features, subject information, and second target CAT-scores. The subject population may especially comprise the target subject (with corresponding subject identifier) and the training data may cover a target subject training timeframe of at least 14 days for the target subject, such as at least 30 days, especially at least 60 days. Further, in embodiments, for at least part of the subject population the training data may cover a timeframe of at least 30 days, such as at least 45 days, especially at least 90 days. In further embodiments, the second training data features and the second data features comprise the same feature types, especially consist of the same feature types.

In further embodiments, the second trained model may (also) consider prior CAT scores. Hence, in embodiments, the second input data may further comprise (second) prior CAT-scores. Similarly, in embodiments, the second training data may include second training prior CAT-scores, especially prior subject-reported CAT-scores.

Although a goal of CAT-score prediction may be to reduce the need for subject to fill out CAT questionnaires, it may be beneficial to occasionally request the subject to fill out a CAT questionnaire to evaluate ongoing model performance. In particular, the predicted CAT-scores may, over time, start to deviate from the subjective CAT-scores a subject would report. Hence, in embodiments, the method may further comprise a subject re-evaluation stage. In such embodiments, the method, especially the subject re-evaluation stage, may comprise periodically requesting the target subject to provide user input to determine subject-reported CAT-scores. For instance, the user input may (directly) comprise a subject-reported CAT-score or may comprise data from which the subject-reported CAT-score can be derived, e.g., answers to the CAT questionnaire. The method, especially the subject re-evaluation stage, may further comprise comparing subject-reported CAT-scores to CAT-score predictions (previously made by the trained model) to determine a prediction performance. Accordingly, the method may comprise recording the CAT-score predictions, especially for later (re-)evaluation. The method, especially the subject re-evaluation stage, may further comprise taking actions when the prediction performance falls below a predefined performance threshold. For instance, when the prediction performance falls below a predefined performance threshold, the method may comprise re-training (or 'adjusting') the trained model using the subject-reported CAT-scores and corresponding data features. Additionally, or alternatively, when the prediction performance falls below a predefined performance threshold, the method may comprise transmitting a performance notification, especially to one or more of the target subject, a data scientist and/or a physician. In particular, the performance notification may indicate that the prediction performance for the target subject has fallen below the predefined performance threshold, the performance notification optionally comprising the subject-reported CAT-score(s) and the CAT-score prediction(s). Based on such information, a physician (or data scientist) may consider whether model re-training would be appropriate.

In further embodiments, the computer-implemented method may further comprise an alert stage. In such embodiments, the method, especially the alert stage, may comprise comparing the CAT-score prediction to a predefined (individualized) alert threshold. If, in such embodiments, the CAT-score prediction exceeds the predefined alert threshold, the method, especially the alert stage, may comprise transmitting an exceedance notification, especially to one or more of the target subject, a data scientist and/or a physician. The exceedance notification may indicate that the CAT-score has exceeded the predefined alert threshold, the exceedance notification optionally comprising the CAT-score prediction and the predefined alert threshold. Based on such information, a physician may consider whether a further evaluation or an intervention would be appropriate. In further embodiments, the exceedance notification may include a request for the target subject to fill out a CAT questionnaire (also see above). Once the target subject fills out the CAT questionnaire, the subject-reported CAT-score may (also) be shared with the physician. Additionally or alternatively, upon receiving the exceedance notification, the physician may trigger a notification requesting the target subject to fill out a CAT questionnaire (on the current date or on a future date). Additionally or alternatively, the exceedance notification may include a recommendation for the subject to consult a physician for an assessment. In particular, the CAT-score prediction may thus serve as an early warning indicator which may be followed by an assessment by the physician. Thereby, a potential progression in the disease state may be caught early and be addressed by the physician.

As described above, the method may comprise providing a CAT-score prediction for the target subject on a target date, wherein the prediction is based on physiological data, a subject identifier, and on one or more of subject-reported prior CAT-scores and predicted prior CAT scores (from a second model).

In embodiments, the method may further comprise a post-processing stage. The method, especially the post-processing stage, may comprise converting the CAT-score prediction into a human-readable format.

In further embodiments, the method may comprise a transmission stage. In such embodiments, the method, especially the transmission stage, may comprise transmitting the CAT-score prediction (in human-readable format) to a user interface for display. For instance, the CAT-score prediction may be displayed in a numeric format or as a color gradient encoding, e.g., with green for a low score and red for a high score, optionally with simultaneous depiction of CAT-scores and CAT-score predictions for multiple days. Accordingly, in embodiments, the method may comprise displaying the CAT-score prediction on the user interface, optionally together with physiological data, and optionally with explanations for the predictions ("explainable AI"). The explanations may comprise feature importance information, e.g. based on SHAP values as described in LUNDBERG SM and SU-IN L., A unified approach to interpreting model predictions, Advances in neural information processing systems 30 (2017), which is hereby herein incorporated by reference, or based on other approaches, e.g. partial dependence (PDP) plots or individual conditional expectation (ICE) plots, with scatter plots for developments of key physiological variables in the subject. The information may especially be presented such that it is intuitive and explorable for the physician.

In a second aspect, the invention may provide a computer-implemented (supervised) training method for providing a trained (machine learning) model, especially a trained regression model. The computer-implemented training method may herein for brevity also be referred to as "training method". The model may especially be trained for providing a CAT-score prediction for a target subject, i.e., the trained model may be configured for providing a CAT-score prediction for a target subject. In embodiments, the training method may comprise a data processing stage, a training stage, and a deployment stage. The training method, especially the data processing stage, may comprise generating training data for a (training) subject population of subjects based on physiological data on the subjects, especially wherein the physiological data comprises electrocardiogram data, temperature data, and motion data. Especially, the subject population may include the target subject. The training data may cover a (training) timeframe of at least 14 days, especially at least 30 days, for the target subject and at least 30 days, such as at least 45 days, especially at least 60 days, for (at least part of) other subjects (of the subject population). For each subject in the subject population, the training data may comprise data features (or 'training data features'), prior CAT-scores (or 'training CAT-scores'), especially prior CAT-score predictions and/or subject-reported CAT-scores, subject information (or 'training subject information'), and target CAT-scores. In embodiments, the data features may comprise (at least) a respiration rate feature, a heart rate variability feature, a heart rate feature, and an activity feature, especially wherein the data features are (pre-)processed into a suitable format for model training. In further embodiments, the prior CAT-scores have numerical values, especially wherein the prior CAT-scores are modelled as continuous numerical data. In further embodiments, the subject information comprises subject identifiers. In particular, each subject in the subject population, including the target subject, may be assigned an (individual) subject identifier. The method, especially the training stage, may comprise training a model (iteratively) based on the training data using a supervised training algorithm in order to provide model parameters (or "model weights"), especially to provide model parameters for CAT-score prediction. Further, the method, especially the deployment stage, may comprise providing a trained model based on the model and the model parameters.

In specific embodiments, the invention provides a computer-implemented training method for providing a trained model, wherein the trained model is configured for providing a CAT-score prediction for a target subject, wherein the computer-implemented training method comprises: (a) generating training data for a subject population of subjects based on physiological data on the subjects, wherein the subject population includes the target subject, wherein the physiological data comprises electrocardiogram data, temperature data, and motion data covering a timeframe of at least 14 days for the target subject; wherein for each subject in the subject population the training data comprises data features, prior CAT-scores, subject information, and target CAT-scores, wherein: the data features comprise a respiration rate feature, a heart rate variability feature, a heart rate feature, and an activity feature; the subject information comprises subject identifiers; (b) training a model based on the training data using a supervised training algorithm in order to provide model parameters; and (c) providing a trained model based on the model and the model parameters.

In particular, the training method may provide a trained model suitable for use in the prediction method. As will be clear to the person skilled in the art, model training and model inference may typically be performed on the same types of features. Hence, the above descriptions of the data features, the prior CAT-scores, and the subject information in the context of the prediction method may analogously apply to the training method.

Further, as described above, the prediction method may comprise the use of a second trained model for predicting CAT-scores concurrent to the data features, and the use of a (first) trained model for providing a CAT-score prediction for a target date based on, amongst others, prior CAT-scores comprising the prior CAT-score predictions (of the second model). In embodiments, the training method may be for providing the (first) trained model, especially for providing a trained model configured to provide a CAT-score prediction for a (future) target date. In further embodiments, the training method may be for providing the second trained model, especially for providing a second trained model configured to provide a concurrent CAT-score prediction.

In specific embodiments, the computer-implemented training method is a computer-implemented supervised training method for providing a trained model, wherein the trained model is a trained regression model, and wherein the trained model is configured for providing a CAT-score prediction for a target subject. The training method may comprise one or more of a data processing stage, a preparation stage, a training stage, and a deployment stage. The computer-implemented training method, especially the data processing stage may comprise generating training data for a (training) subject population of (training) subjects based on physiological data on the subjects. The subject population may include the target subject. Especially, the physiological data comprises electrocardiogram data, temperature data, and motion data covering a training timeframe of at least 14 days for the target subject, and especially at least 30 days for other subjects (in the subject population). In embodiments, for each subject in the subject population the training data comprises data features, prior CAT-scores, especially prior model predictions and/or subject-reported CAT-scores, subject information, and target CAT-scores. In further embodiments, the data features may comprise (at least) a respiration rate feature, a heart rate variability feature, a heart rate feature, and an activity feature, and the subject information may comprise subject identifiers. The training method, especially the preparation stage, may comprise (a) dividing the training data into a training subset and a testing subset and (b) loading a model, wherein model parameters of the model comprise initial parameter values. Further, the training method, especially the training stage, may comprise training the model based on the training data using a supervised training algorithm, especially wherein the training comprises executing an iterative training cycle for a predefined number of iterations or until a performance criterion is met (such as a performance threshold being met or such as overfitting being detected). In embodiments, the training cycle may comprise a training substage and an evaluation substage. In particular, the training substage may comprise training the model using the supervised training algorithm based on the training subset in order to update the model parameters, for instance based on a predefined loss function. The evaluation substage may comprise evaluating performance of the model based on the testing subset to determine a (prediction) performance of the model and updating the model parameters based on the (prediction) performance. After the training stage, the method, especially the deployment stage, may comprise providing a trained model based on the model and the (updated) model parameters.

The training method may be a supervised training method. Supervised machine learning is a type of machine learning where the model is trained with training data including the target output (or "correct answers"). In particular, in the context of the invention, the training method may comprise training the model with training data including target CAT-scores - typically subject-reported CAT-scores - as target output. The objective of supervised learning is to provide a trained model that can predict the output for new, unseen data based on the patterns it learned during training.

As described above, the CAT-score may be strongly autocorrelated, and recent CAT-scores may thus be informative for predicting a CAT-score for a moment in the near future. Therefore, the training data may specifically include both prior CAT-scores and target CAT-scores. In particular, the term "target CAT-score" may refer to a to-be predicted value for the model, i.e., these are the "correct answers" for model training, whereas the "prior CAT-scores" correspond to CAT-scores for one or more days preceding the (respective) target CAT-score, i.e., the prior CAT-scores are included in the data features from which the model is trained to predict the target CAT-scores.

During training, the (supervised) training algorithm may provide and update model parameters (or "model weights"). The term "model parameter" may herein refer to a value (or 'setting') that can be altered (or "optimized") during model training to improve the performance of the model.

In embodiments, the subject population may comprise the target subject. In particular, as described in the context of the prediction method, the inclusion of training data on the specific target subject may substantially improve the performance of the model with respect to CAT-score predictions for the target subject. Further, the subject population may comprise ≥ 20 other subject, such as ≥ 30 other subjects, especially ≥ 50 subjects. Especially, the training data may cover a (training) timeframe of at least 30 days for the other subjects, such as a timeframe of at least 45 days, especially of at least 60 days. In particular, for each subject (in the subject population) may apply that the training data comprises a plurality of timepoints, wherein the data features and the prior CAT-scores, such as the subject-reported CAT-scores, include (optionally partly imputed) values for each of the timepoints.

It will be clear to the person skilled in the art that various (supervised) training algorithms exist for providing a trained model. For instance, the supervised training algorithm may be selected from the group comprising a gradient boosting algorithm, such as XGBoost, lightGBM, and Catboost, a random forest algorithm, a neural network (NN) algorithm, such as a NN algorithm capable of handling time-series data such as LSTM, 1D CNN, GRU, RNN, TCN, TimesNet, BiTCN, DeepAR, NBEATS, NBEATSx, NHITS, TiDE, DeepNPTS, TSMixer, TSMixerx, MLPMultivariate, DLinear, NLinear, TFT, Informer, Autoformer, FedFormer, PatchTST, iTransformer, StemGNN, TabFPN, Chronos, AutoGluon and Time-LLM.

In further embodiments, the supervised training algorithm may be selected form the group consisting of XGBoost, Catboost, lightGBM, LSTM, 1D CNN, GRU, NHITS, Informer, Autoformer, and Time-LLM, preferably XGBoost, or preferably Catboost, or preferably LSTM, or preferably 1D CNN. Particularly good results were obtained with XGBoost, Catboost, LSTM, 1D CNN, and mixed LSTM-1DCNN models.

Hence, in embodiments, the supervised training algorithm may be a gradient boosting algorithm. Especially, the supervised training algorithm may comprise XGBoost, such as described in CHEN T and GUESTRIN C, XGBoost: A Scalable Tree Boosting System, Proceedings of the 22nd ACM SIGKDD International Conference on Knowledge Discovery and Data Mining, 2016, pages 785-794, which is hereby herein incorporated by reference. In further embodiments, the supervised training algorithm may comprise lightGBM, such as described in KE G, et al., LightGBM: A Highly Efficient Gradient Boosting Decision Tree, Advances in Neural Information Processing Systems, 2017, which is hereby herein incorporated by reference. In further embodiments, the supervised training algorithm may comprise Catboost, as described in PROKHORENKOVA L, et al. "Catboost: unbiased boosting with categorical features." Advances in neural information processing systems 31 (2018), which is hereby incorporated by reference.

In further embodiments, the supervised training algorithm may comprise a neural network algorithm. Especially, the supervised training algorithm may comprise 1D CNN, such as described in LECUN Y, et al., Gradient-based learning applied to document recognition, Proceedings of the IEEE, 86(11), 2278-2324, and in KIRANYAZ S, et al., 1D convolutional neural networks and applications: A survey, Mechanical systems and signal processing 151, 2021, which are hereby herein incorporated by reference. In further embodiments, the supervised training algorithm may comprise GRU, such as described in CHO K, et al., Learning Phrase Representations using RNN Encoder-Decoder for Statistical Machine Translation, Proceedings of the 2014 Conference on Empirical Methods in Natural Language Processing (EMNLP), 2014, pages 1724-1734, which is hereby herein incorporated by reference. In further embodiments, the supervised training algorithm may comprise NHITS, such as described in CHALLU C, et al., NHITS: Neural Hierarchical Interpolation for Time Series Forecasting, Proceedings of the AAAI Conference on Artificial Intelligence, 37(6), 2023, which is hereby herein incorporated by reference. In further embodiments, the supervised training algorithm may comprise Informer, such as described in ZHOU C. et al., Informer: Beyond efficient transformer for long sequence time-series forecasting, Proceedings of the AAAI Conference on Artificial Intelligence, pages 11106-11115, 2021, which is hereby herein incorporated by reference. In further embodiments, the supervised training algorithm may comprise Autoformer, such as described in WU H. et al., Autoformer: Decomposition transformers with auto-correlation for long-term series forecasting, Advances in Neural Information Processing Systems, 34, pages 22419-22430 2021, which is hereby herein incorporated by reference. In further embodiments, the supervised training algorithm may comprise Time-LLM, such as described in JIN M. et al., Time-LLM: Time Series Forecasting By Reprogramming Large Language Models, arXiv preprint arXiv:2310.01728, which is hereby herein incorporated by reference. In further embodiments, the supervised training algorithm may comprise TimeGPT, as referenced in GARZA A et al, TimeGPT-1., arXiv preprint arXiv:2310.03589 (2023), which is hereby incorporated by reference.

In embodiments, the training method may further comprise a data collection stage. In such embodiments, the training method, especially the data collection stage, may comprise acquiring, such as receiving, the physiological data (on the subject population), such as from a data repository, or such as from a sensor arrangement.

In a further aspect, the invention may provide a data processing system (or "control system" or "computer system") comprising means for carrying out the steps of the prediction method of the invention and/or of the training method of the invention, especially at least the prediction method, or especially at least the training method. In particular, in embodiments, the data processing system may be configured to execute the prediction method (using a trained model). In further embodiments, the data processing system may be configured to execute the training method.

In a further aspect, the invention may provide a system for providing a CAT-score prediction, especially for a target subject. The system may comprise data processing system of the invention and a sensor arrangement. In particular, the sensor arrangement may comprise an electrocardiogram sensor, a temperature sensor, and a motion sensor, and the sensor arrangement may (be configured to) determine (or 'acquire' or 'sense') physiological data of a target subject and to provide the physiological data to the data processing system. The physiological data may comprise electrocardiogram data, temperature data, and motion data. Hence, the sensor arrangement may comprise: (a) an electrocardiogram sensor configured to determine electrocardiogram data on the target subject, (b) a temperature sensor configured to determine temperature data on the target subject, and (c) a motion sensor configured to determine motion data on the target subject.

The sensor arrangement and the data processing system may especially be physically separated. For instance, the sensor arrangement may be arranged in proximity to a target subject, whereas the data processing system may be arranged remote from the target subject, such as at a dedicated site for executing the prediction method. The sensor arrangement may be configured to provide the physiological data to the data processing system, e.g., by (wirelessly) transmitting the physiological data to the data processing system, such as, for example, via a WiFI connection or via an Internet connection.

In embodiments, the system may comprise a wearable device, especially wherein the wearable device comprises the sensor arrangement. In particular, the sensor arrangement may be configured to determine the physiological data of the target subject when the target subject is wearing the wearable device. Such configurations may be particularly suitable for consistent collecting of the physiological data as the sensor arrangement may continue to track the physiological data of the subject as the subject moves around. By contrast, a home monitoring system may typically be incapable of tracking physiological data of the subject when the subject leaves their home.

In further embodiments, the system, especially the wearable device, may comprise a user interface. In particular, the user interface may be configured to receive user input from the target subject and/or from a physician. The user interface may further be configured to display information, such as to the target subject and/or the physician. For instance, in embodiments, the user interface may be configured for having the target subject take a CAT questionnaire, i.e., the user interface may display the questions of the CAT questionnaire and may be configured to receive the subject's responses. Thereby, a subject-reported CAT-score may be conveniently acquired.

In further embodiments, the system may comprise an implantable device, especially wherein the implantable device comprises the sensor arrangement. In particular, the sensor arrangement may be configured to determine the physiological data of the target subject when the implantable device is implanted in the subject. Such configurations may be particularly suitable for consistent collecting of the physiological data as the sensor arrangement may continue to track the physiological data of the subject as the subject moves around.

In a further aspect, the invention may provide a computer program product. The computer program product may comprise instructions for execution on a data processing system (or "computer system"), wherein the instructions, when executed by the data processing system, cause the data processing system to carry out the prediction method or the training method, especially the prediction method, or especially the training method.

In a further aspect, the invention may provide a data carrier. The data carrier may comprise instructions for execution on a data processing system (or "computer system"), wherein the instructions, when executed by the data processing system, cause the data processing system to carry out the prediction method or the training method, especially the prediction method, or especially the training method.

In a further aspect, the invention may provide the trained model as such. In particular, the invention may provide the trained model as provided using the training method of the invention.

In embodiments, the trained model may be configured to (a) receive input data on a target subject, wherein the input data comprises data features, prior CAT-scores, and subject information, wherein the data features comprise (at least) a respiration rate feature, a heart rate variability feature, a heart rate feature, and an activity feature, wherein the prior CAT-scores precede a target date, and wherein the subject information comprises a subject identifier; and (b) to provide a CAT-score prediction for the target subject on the target date based on the input data.

In further embodiments, the trained model may be configured to provide a CAT-score prediction for a future target date. In particular, the timeframe (for the data features and the prior CAT-scores) may include a last date, and the target date may be selected from the range of 1 - 14 days after the last date, such as 2-10 days after the last date, especially 5-7 days after the last date.

In further embodiments, the trained model may be configured to provide a CAT-score prediction for a concurrent target date, especially for one or more target dates within a timeframe corresponding to the input data, especially within a timeframe corresponding to the data features.

In a further aspect, the invention may provide a data carrier carrying thereupon the trained model according to the invention.

### BRIEF DESCRIPTION OF THE DRAWINGS

Embodiments of the invention will now be described, by way of example only, with reference to the accompanying schematic drawings in which corresponding reference symbols indicate corresponding parts, and in which: Fig. 1A-C schematically depict embodiments of the computer-implemented method. Fig. 2 schematically depicts an embodiment of the system. Fig. 3A-B schematically depict experimental results obtained using the approach of the invention. The schematic drawings are not necessarily on scale.

### DETAILED DESCRIPTION OF THE EMBODIMENTS

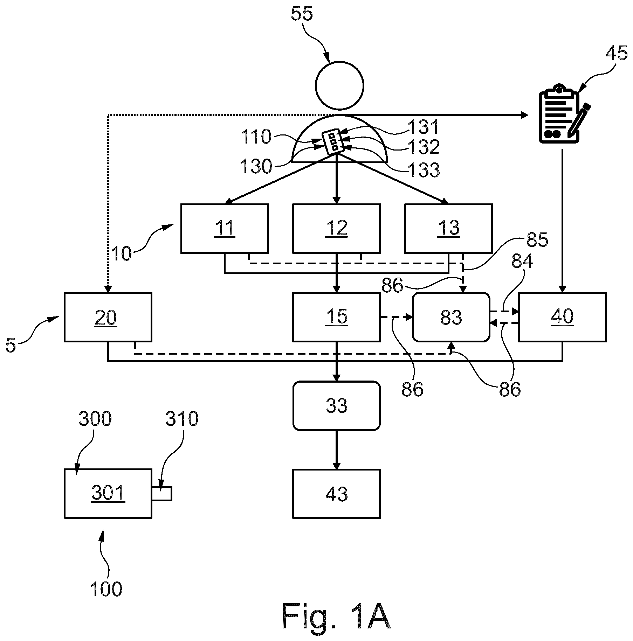

Fig. 1A schematically depicts an embodiment of the computer-implemented (inference) method for providing a CAT-score prediction 43 for a target subject 55 and a target date. In the depicted embodiment, the target subject 55 has associated subject information 20. Further, the target subject 55 is wearing a wearable device (110), which comprises a sensor arrangement 130 configured to provide physiological data 10. Specifically, the sensor arrangement 130 comprises an electrocardiogram sensor 131 providing electrocardiogram data 11, a temperature sensor 132 providing temperature data 12, and a motion sensor 133 providing motion data 13. Based on the physiological data 10, data features 15 are generated. In the depicted embodiment, the target subject 55 further provides a subject-reported CAT-score 45, such as by responding to the CAT questions. The subject-reported CAT-scores 45 and/or second CAT-score predictions 84 (from a second model 83) make up prior CAT-scores 40. Together, the subject information 20, the data features 15, and the prior CAT-scores 40 make up the user input 5, which is fed to a trained model 33, wherein the trained model 33 is configured to provide the CAT-score prediction 43 based on the input data 5.

In particular, Fig. 1A schematically depicts a computer-implemented (inference) method (or "prediction method") for providing a CAT-score prediction 43 for a target subject 55 and a target date using a trained model 33. In the depicted embodiment, the prediction method comprises (at least) a data processing stage and an inference stage. The prediction method, especially the data processing stage, comprises generating (or "extracting") the data features 15 from the physiological data 10 on (or "from") the target subject 55, especially wherein the physiological data 10 comprises electrocardiogram data 11, temperature data 12, and motion data 13. The data features 15 may especially comprise (at least) a respiration rate feature, a heart rate variability feature, a heart rate feature, and an activity feature. The data processing stage may further comprise (pre-)processing the data features 15 into a suitable format for the trained model 33. The prediction method, especially the inference stage, may comprise providing input data 5 to the trained model 33 and generating, by the trained model 33, the CAT-score prediction 43 based on the input data 5. In particular, the input data 5 may comprise the data features 15, prior CAT-scores 40 for the target subject 55, and target subject information 20. In embodiments, the prior CAT-scores 40 may (correspond to timepoints that) precede the target date. Further, in embodiments, the target subject information 20 may comprise a subject identifier (code) for the target subject 55. In embodiments, the prediction method may further comprise (post)processing the CAT-score prediction 43 into a human-readable format and transmitting the (postprocessed) CAT-score prediction 43 to a user interface 301 for display.

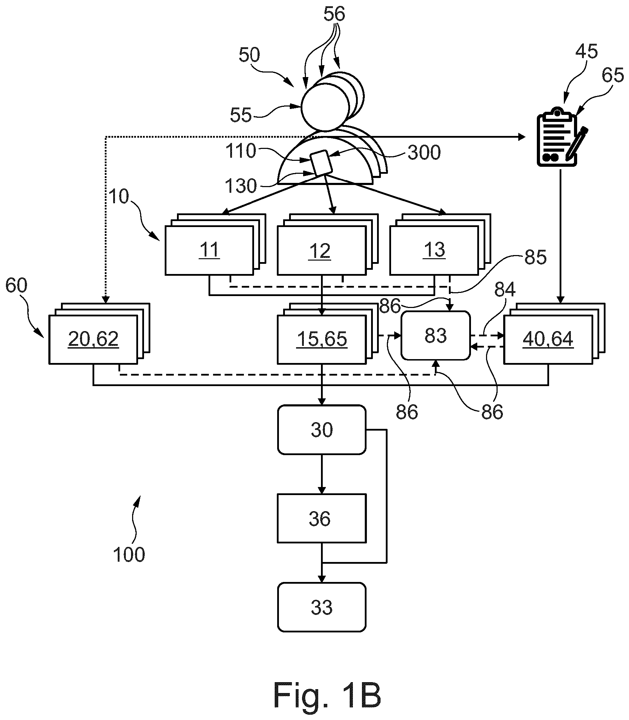

The prior CAT-scores 40 may especially comprise subject-reported CAT-scores 45 and/or second CAT-score predictions 84. In particular, during training of the model (also see Fig. 1B and the corresponding description), subject-reported CAT-scores 45 may generally be available, but during a subsequent operational period of the trained model 33, subject-reported CAT-scores 45 may not be consistently available. Surprisingly, the inventors have found that concurrent CAT-score predictions - i.e., for timepoints, e.g. days, for which physiological data is available - may supplement or replace subject-reported CAT-scores for providing CAT-score predictions for future target dates. Hence, in embodiments, the prediction method may comprise a second data processing stage and a second inference stage. The prediction method, especially the second data processing stage, may comprise generating second data features 85 from the physiological data, wherein the second data features 85 comprise a respiration rate feature, a heart rate variability feature, a heart rate feature, and an activity feature. The prediction method, especially the second inference stage, may further comprise providing second input data 86 to a second trained model 83, and generating, by the second trained model 83, at least part of the prior CAT-scores 40, especially the second CAT-score predictions 84. Accordingly, the second trained model 83 may (be configured to) provide second CAT-score predictions 84 based on the second input data 86, especially based on (at least) the second data features 85. In embodiments, the second input data 86 comprises one or more of the target subject information 20, the second data features 85 and prior CAT-scores (preceding the target date for the second trained model 83).

In embodiments, the data features 15 and the second data features 85 may be the same. However, in alternative embodiments, the data features 15 and the second data features 85 may differ. In particular, some data features may be particularly informative for concurrent CAT-score predictions (for the same day(s) for which the physiological data is available), whereas other data features may be particularly informative for CAT-score progression over time, and, by extent, for the CAT-score a few days into the future.

The prediction method may, in embodiments, further comprise a data collection stage. In such embodiments, the prediction method, especially the data collection stage, may comprise acquiring (such as "receiving") the physiological data 10 on the subject 55 from a sensor arrangement 130.

Fig. 1A further schematically depicts an embodiment of a trained model 33 for providing a CAT-score prediction 43 for a target subject 55 on a target date. In particular, the trained model 33 may be configured for receiving input data 5 on a target subject 55, wherein the input data 5 comprises data features 15, prior CAT-scores 40, and subject information 20, and to provide a CAT-score prediction 43 for the target subject 55 on the target date based on the input data 5.

Fig. 1A further schematically depicts an embodiment a data processing system 300 comprising means for carrying out the steps of the computer-implemented method according to the invention. In further embodiments, the data processing system 300 may (also) be configured to execute the computer-implemented training method of the invention.

Specifically, Fig. 1A schematically depicts an embodiment of a system 100 for providing a CAT-score prediction 43, wherein the system 100 comprises the data processing system 300 and the sensor arrangement 130. In particular, the sensor arrangement 130 comprises an electrocardiogram sensor 131, a temperature sensor 132, and a motion sensor 133, and is configured to determine the physiological data 10 of the target subject 55, and to provide the physiological data to the data processing system 300. The physiological data 10 may comprise electrocardiogram data 11, temperature data 12, and motion data 13.

In the depicted embodiment, the system 100 comprises a wearable device 110, wherein the wearable device 110 comprises the sensor arrangement 130, and wherein the sensor arrangement 130 is configured to determine the physiological data 10 of the target subject 55 when the target subject 55 is wearing the wearable device 110. In particular, the wearable device 110 is functionally couplable to the data processing system 300.

The system 100, especially the data processing system 300, may comprise a user interface 301, wherein the user interface 301is configured to receive user input from the target subject 55 and/or from a physician, i.e., the data processing system 300 may be configured to receive user input via the user interface 301. For instance, the system 100, especially the user interface 300, may be configured for receiving a subject-reported CAT-score 45 from the subject 55.

Fig. 1A further schematically depicts a data carrier 310. In embodiments, the data carrier 310 may carry thereupon program instructions which, when executed by a data processing system 300, cause the data processing system 300 to carry out the computer-implemented method of the invention and/or the computer-implemented training method of the invention. In further embodiments, the data carrier may carry thereupon the trained model of the invention.

Fig. 1B schematically depicts an embodiment of the computer-implemented training method for providing a trained model 33 for providing a CAT-score prediction 43 for a target subject. As depicted, the training method involves data from a subject population 50 of a plurality of subjects 56 including the target subject 55. In the depicted embodiment, training data 60 comprises (a) subject information 20, data features 15 and prior CAT-scores 40 for each subject 56 in the subject population 50. Based on the training data, a model 30 is trained using a supervised training algorithm in order to provide model parameters 36, and a trained model 33 is provided based on the model 30 and the model parameters.

In particular, Fig. 1B schematically depicts an embodiment of the computer-implemented (supervised) training method for providing a [machine learning] trained [regression] model 33, wherein the trained model 33 is configured for providing a CAT-score prediction 43 for a target subject 55, wherein the computer-implemented training method comprises a data processing stage, a training stage and a deployment stage. The training method, especially the data processing stage, comprises generating training data 60 for a (training) subject population 50 of subjects 56 based on physiological data 10 on the subjects 56, especially wherein the physiological data 10 comprises electrocardiogram data 11, temperature data 12, and motion data 13. The subject population 50 may especially include the target subject 55. The training data may cover a (training) timeframe of at least 7 days for the target subject 55, such as at least 14 days, especially at least 30 days. Further, in embodiments, the training data may cover a (training) timeframe of at least 30 days for (at least part of the) other subjects 56, such as at least 45 days, especially at least 60 days. In particular, for each subject 56 in the subject population 50 the training data 60 may comprise data features 15 (or "training data features" 65), prior CAT-scores 40 (or "training CAT-scores" 64), such as second CAT-score predictions 84 and/or subject-reported CAT-scores 45, and subject information 20 (or "training subject information" 62), the subject information 20 especially including subject identifiers (for each subject 56). In embodiments, the data features 15 may comprise (at least) a respiration rate feature, a heart rate variability feature, a heart rate feature, and an activity feature, especially (pre-)processed into a suitable format for the model 30 and/or the (supervised) training algorithm. The training method, especially the training stage, comprises training a model 30 (iteratively) based on the training data 60 using a supervised training algorithm in order to provide model parameters 36 (for CAT-score prediction 43). Subsequently, the method, especially the deployment stage, comprise providing a trained model 33 based on the model 30 and the model parameters 36.

For visualization purposes, a subject population 50 of three subjects 56, including the target subject 55, is depicted in Fig. 1B. For each depicted subject 56, corresponding physiological data 10 and training data 60 is schematically depicted. In general, however, there may be substantially more than three subjects 56 in the subject population 50. For instance, in embodiments, the subject population 50 comprises the target subject 55 and at least 20 other subjects 56, such as at least 30 other subjects, especially at least 60 other subjects.

In further embodiments, for each subject 56 (in the subject population 50), the training data 60 may comprise a plurality of timepoints, wherein the data features 15 and the prior CAT-scores 40, such as the subject-reported CAT-scores 45, include (optionally partly imputed) values for each of the timepoints (for the corresponding subject 56). As will be clear to the person skilled in the art, the timepoints (e.g., dates) may differ for different subjects 56.

In embodiments, the training method further comprises a data collection stage. The method, especially the data collection stage, may comprise acquiring the physiological data 10 on the subject population 50.

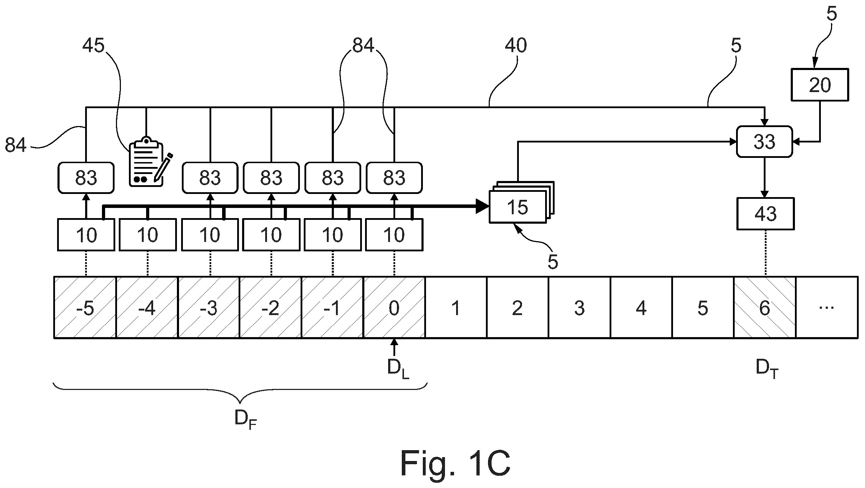

Fig. 1C schematically depicts a further embodiment of the computer-implemented prediction method. In the depicted embodiment, physiological data 10 is available for a timeframe D_{F} covering day 0 - the last date D_{L} - and the preceding days -1 to -5, whereas the target date D_{T} for the CAT-score prediction is day 6. For instance, day 0 may be the present day and day 6 may be six days into the future. For day 0 to day -5, physiological data 10 is available, and for day -4, also a subject-reported CAT-score 45 is available. For each day in the timeframe D_{F}, the physiological data 10 is converted to data features 15. Solely for visualization purposes, the data features 15 are depicted above day 2. For days -5, -3, -2, -1, and 0, the physiological data 10 are used together with a second trained model 83 to generate a second CAT-score prediction 84 (or "concurrent CAT-score prediction"). Specifically, in the depicted embodiment, only the physiological data 10 of a given day is used to generate the second CAT-score prediction 84 for that day, e.g., the physiological data for day -5 is used to provide the second CAT-score prediction 84 for day -5. The second CAT-score predictions 84 and the subject-reported CAT-score for day -4 are combined to provide the prior CAT-scores 40. The data features 15, the prior CAT scores 40 and the subject information 20 are subsequently fed to the trained model 33 to generate a CAT-score prediction 43 for day 6. Alternatively, despite a subject-reported CAT-score 45 for day -4 being available, a second CAT-score prediction 84 may be used instead.

Hence, in the depicted embodiment, the physiological data 10 and the prior CAT-scores 40 correspond to a timeframe of n days, wherein n=6, wherein the timeframe includes a last date, and wherein the target date is selected to be six days after the last date. Accordingly, in embodiments, the prediction method may comprise providing a CAT-score prediction for a future target date, e.g. 1-14 days, such as 5-7 days, in the future, based on physiological data and prior CAT-scores of the current day and previous days.

Further, in the depicted embodiment, the input data 5 may comprise a plurality of timepoints (or "observations"), such as one timepoint for each of day 0, -1, -2, -3, -4, and - 5, wherein for each timepoint the input data 5 includes (optionally partly imputed) values for the data features 15 and for the prior CAT-scores 40.

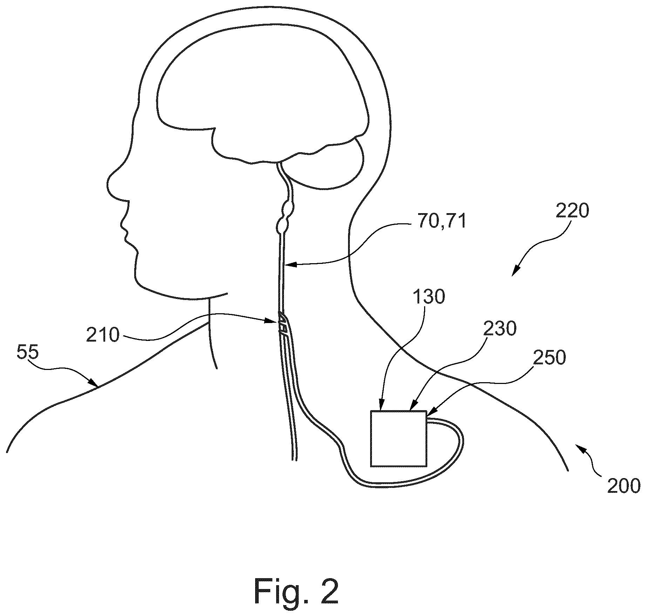

Fig. 2 schematically depicts an implantable arrangement 200 comprising an (implantable) device 220, wherein the implantable device 220 is implanted in the target subject 55, especially (at least partly) at the neck of the target subject 55. In the depicted embodiment, the implant arrangement 200 comprises an electrode arrangement 210, an implant control system 230, and a pulse generator 250. The electrode arrangement 210 is configured to engage a nerve 70, especially a vagus nerve 71, of the target subject. The implant control system 230 is configured to control the pulse generator 250. The pulse generator 250 is configured to provide an electrical pulse to the nerve via the electrode arrangement 210, e.g., to modulate action potential conduction in the nerve. Such vagus nerve stimulation devices may, for instance, be suitable for blocking parasympathetic pulmonary B-fibers which drive bronchoconstriction and mucus secretion in COPD patients. Such implants may be particularly suitable for integration of the sensor arrangement 130, as depicted in Fig. 2, such that various device functionalities relevant to a COPD patient are conveniently combined in a single device, which may reduce the burden on the patient and the number of interventions from a physician. For instance, the sensor arrangement may be integrated with the implantable pulse generator (or "IPG"), where for example the motion sensor 133 (e.g., the accelerometer) can be inside the housing (of the IPG), the electrocardiogram sensor (e.g., the ECG electrodes) can be outside the housing, and where the temperature sensor can be inside and/or outside of the housing, especially at least inside of the housing, or especially at least outside of the housing.

Hence, in a further aspect, the invention may provide an implantable device 220, wherein the implantable device 220 comprises the sensor arrangement 130, and wherein the sensor arrangement 130 is configured to determine physiological data 10 of the target subject 55 when the implantable device 220 is implanted in the target subject 55.

In specific embodiments, the implantable device 220 may comprise an action potential modulation device, such as a vagus nerve stimulation device. In particular, the implantable device 220 may be configured to provide an electrical signal to a nerve to modulate action potential conduction in the nerve, such as to inhibit (or "block") action potential conduction in the nerve, or such as to stimulate action potential conduction in the nerve, especially to provide a target physiological response.

In further embodiments, the implantable device 220 may comprise or be functionally couplable to a data processing system 300, wherein the sensor arrangement 130 is configured to provide the physiological data 10 to the data processing system 300, and wherein the data processing system 300 is configured to provide the CAT-score prediction based on the physiological data 10 using the method of the invention. In further embodiments, the data processing system 300 may be configured to control the sensor arrangement 130 based on the CAT-score prediction, especially based on one or more predefined CAT-score thresholds, e.g., predefined by a physician.

### Experiments

A multicenter, prospective cohort study was conducted at 11 sites in Germany. Between 05-12-2022 and 25-08-2023, 77 participants (67 participants in the COPD cohort and 10 participants in the calibration cohort) were screened and included in the study. Main inclusion criteria required participants with COPD to be between 40 and 80 years old, capable of providing informed consent, diagnosed with COPD stage II to IV, and having a history of moderate or severe exacerbations. Exclusion criteria for COPD participants included the likelihood of not completing the study, having serious concurrent medical conditions, possessing implanted electronic devices, or having allergies to silicon or hydrogel.

High compliance of the participants facilitated collection of large amounts of data (125,560 hours of sensor data, 5,627 CAT questionnaires and 48,396 activities labeled). 92% of the COPD participants wore the device on average ≥18 hours/day and 92% also completed the CAT questionnaire in ≥ 80% of days and 89% labeled ≥ 5 activities/day on average.

Unless explicitly stated otherwise, the experiments described herein are described using the following materials and methods.

Sensor arrangement - The Vivalink (51 E Campbell Ave, Campbell, California 95008, US) wearable patch device was used to collect physiological data on subjects. The model of the Vivalink wearable device used in this study was a CE marked device (model VV-330) modified to add a temperature measurement algorithm (a software modification).

Study design and conduction - The multicenter, prospective cohort study was conducted at 11 sites in Germany. A cohort of participants with COPD Global Initiative for Chronic Obstructive Lung Disease (GOLD) stage II-IV was followed for 3 months (observation period). During a baseline visit each enrolled participant was provided with the Vivalink wearable patch device which recorded ECG, temperature and accelerometer data, and with a connected smartphone. A digital application was provided with the smartphone for the participants to fill in a daily CAT questionnaire, to label activities and events relevant for the interpretation of sensor signals, and to receive notifications/reminders. At the end of the 3-month observation period, the participants returned the devices during a regular COPD related visit scheduled at 3 months (± 14 days) after the baseline visit (Day 0).

Statistical methods - Standard analyses and descriptive statistics were carried out using R 4.2 or higher (The R Foundation for Statistical Computing, Vienna, Austria). Linear and mixed models were fit using SAS 9.4 or higher, or JMP 17.0 (SAS Institute Inc., Cary, NC, USA) or using R 4.2 or higher (The R Foundation for Statistical Computing, Vienna, Austria). Machine learning models were implemented using Python 3.8 or higher (The Python Software Foundation, Wilmington, Delaware, USA).

Feature generation - Heart rate (HR) and heart rate variability (HRV) features - The raw electrocardiogram (ECG) signal was put through denoising algorithms, where in short (i.e. 2 minute) windows the signal was analyzed to remove stretches that could not be used for further analysis by checking for variance, zero values, and missing values. The denoised signal was then filtered using a bandpass filter and a baseline wander filter. Additional thresholding filters were applied to remove any signal outside expectation. A peak finding algorithm was run along the signal. The identified peaks were used as the basis for the calculation of HR and HRV features. Based on the identified peaks, heart rate and stress index were calculated. Further, mean, median, mode, std, min and max per day were calculated for HR. Additionally, the following data features corresponding to percentages of heart rate above or below predefined thresholds were calculated: HR below mean; HR above mean; HR < 50; HR > 80; and 50 <HR < 80.

Feature generation - Respiration Rate (RR) - Respiration rate features were determined analogously to HR and HRV features. Specifically, the raw electrocardiogram (ECG) signal was put through denoising algorithms, where in short (i.e. 2 minute) windows the signal was analyzed to remove stretches that could not be used for further analysis by checking for variance, zero values, and missing values. The denoised signal was then filtered using a bandpass filter and a baseline wander filter. Additional thresholding filters were applied to remove any signal outside expectation. A peak finding algorithm was run along the signal. The identified peaks were used as the basis for the calculation of RR features. Root Mean Square of Successive Differences (RMSSD), Number of pairs of successive NN intervals differing by more than 50 ms (NN50), Percentage of successive NN intervals differing by more than 50 ms (PNN50), Very Low Frequency power (VLF), Low Frequency power (LF), High Frequency power (HF) and respiration rate were calculated using the python packages heartpy and neurokit. Based on these data, also the derived inspiration expiration ratio was calculated. Further, mean, mode, min and max per day were calculated for RR.

Feature generation - step count - the accelerometer data was transformed using a median filter and a frequency filter. The filtered data was then used to calculate the magnitude, from which subsequently the peaks were extracted. The peak count was taken to represent the number of steps.

Feature generation - temperature - the temperature data was filtered to remove the first 15 minutes from the data after any gap in wearing the wearable patch device. For each subject, instances of significant increase or decrease of temperature were calculated based on daily mean and standard deviation of the temperature measurements (mean +- 2*std). Additionally, based on the mean of the temperature per subject during the first month, similarity measures were calculated to see how a specific day differs from the mean, using curve similarity measures such as partial curve mapping, area, Frechet distance, curve length, time warping, mean absolute error, and mean squared error. Further, mean, median, mode, std, min and max per day were calculated for temperature.

Feature generation - sleep - an activity index was determined based on motion data, especially accelerometer data. After the activity index is calculated, the mean +-2*standard deviations is calculated, and the data is classified to higher than and lower than those limits. The temperature and heart rate are also used to classify data to larger or smaller than the mean per day. A weighted combination of the 3 classifications was used to determine if a data point is considered as activity or not, based on a given threshold. Only periods of rest larger than 25 minutes were considered.

Feature generation - accelerometer clusters - using the three different axes from the accelerometer data as features, a K-Means clustering algorithm was trained to divide each data point into 6 clusters, using as training data 1% of all possible days/patients. This algorithm was then used to classify the accelerometer data per patient per day, and the counts of elements in each cluster are the resulting features.

Feature generation - activities - the data from 10 volunteers, that used the sensor while following a list of tasks and logging the time of start and end of said task, was used to train a Gradient Boosting regressor to classify activity data. Features were created from accelerometer data and HR data, using typical statistics as mean, median, kurtosis, entropy, skewness, and frequency domain statistics, and PCA. The available data was randomly divided into 80% training data and 20% testing data, ensuring all types of features were included in both sets. The Gradient Booster regressor results were rounded to the nearest integer and then scored using a confusion matrix. The random selection was repeated 10 times, and the scores averaged to 89% accuracy in the classification. The algorithm was then stored and run for each patient each day in 10 second windows, giving as result the amount of time per day classified in each activity.

Feature generation - Chest Dynamics Metric - the data from 10 volunteers, that used the sensor while intentionally coughing was used to train a Gradient boosting regressor. The same features as for the activity classification were used, and the same procedures. An average accuracy of 92% was achieved.

Feature generation - derived features - Minimum, maximum and difference data features were generated based on the past 7 days. Average or slope of change features were generated based on the past 4 days.

An overview of the generated features is provided in table 1:

| **Feature type - name** | **Description** | **Physiological data** |
|---|---|---|
| Activity - Number of steps | Number of steps per day | Accelerometer |
| Activity - Movement cluster - 0 | Belongs to given accelerometer cluster | Accelerometer |
| Activity - Movement cluster - 1 | Belongs to given accelerometer cluster | Accelerometer |
| Activity - Movement cluster - 2 | Belongs to given accelerometer cluster | Accelerometer |
| Activity - Movement cluster - 3 | Belongs to given accelerometer cluster | Accelerometer |
| Activity - Movement cluster - 4 | Belongs to given accelerometer cluster | Accelerometer |
| Activity - Movement cluster - 5 | Belongs to given accelerometer cluster | Accelerometer |
| Activity - Steps (mean per hour) | Steps per hour (mean) | Accelerometer |
| Activity - Sleep Disturbances - count | Total number of Sleep disturbances | ECG, Accelerometer, Temperature |
| Activity - Sleep Disturbances - count - slope4 | 4 day slope of named feature | ECG, Accelerometer, Temperature |
| Activity - Sleep Disturbances - mean duration | Mean duration of sleep disturbances | ECG, Accelerometer, Temperature |
| Activity - Sleep hours | Number of sleep hours | ECG, Accelerometer, Temperature |
| Activity - Sleep hours - normalized | Normalized version of named feature | ECG, Accelerometer, Temperature |
| Activity - Sleep hours - slope4 | 4 day slope of named feature | ECG, Accelerometer, Temperature |
| Activity - Stair climbing (mins) | Going up or down stairs | ECG, Accelerometer, Temperature |
| Activity - stair climbing (mins) - slope4 | 4 day slope of named feature | ECG, Accelerometer, Temperature |
| Activity - Time active | Total time of activity | ECG, Accelerometer, Temperature |
| Activity - Walking (mins) | Walking | ECG, Accelerometer, Temperature |
| Activity - walking (mins) - slope4 | 4 day slope of named feature | ECG, Accelerometer, Temperature |
| Cough - Chest Dynamics Metric | Cough frequency proxy, predicted by ML | ECG, Accelerometer, Temperature |
| Cough - Chest Dynamics Metric - normalized | Normalized cough frequency proxy inside patient | ECG, Accelerometer, Temperature |
| Cough - Chest Dynamics Metric - slope4 | 4 day slope of named feature | ECG, Accelerometer, Temperature |
| Heart Rate - HR above 80 (%) | Percentage of time where HR > 80 | ECG |
| Heart Rate - HR above 80 (%) - slope4 | 4 day slope of named feature | ECG |
| Heart Rate - HR below 50 (%) | Percentage of time where HR < 50 | ECG |
| Heart Rate - HR below 50 (%) - slope4 | 4 day slope of named feature | ECG |
| Heart Rate - HR below daily mean (%) | Percentage of time where HR is below mean | ECG |
| Heart Rate - HR normal (%) | Percentage of time where 50 ≤ HR ≤ 80 | ECG |
| Heart Rate - mean Kubios | Mean heart rate as calculated by Kubios | ECG |
| Heart Rate - mean Kubios - normalized | Normalized version of named feature | ECG |
| Heart Rate - mean Kubios - slope4 | 4 day slope of named feature | ECG |
| Heart Rate - median Kubios - slope4 | 4 day slope of median hart rate as calculated by Kubios | ECG |
| HRV - High Frequency Power | High frequency from ECG | ECG |
| HRV - High Frequency Power - normalized | Normalized version of named feature | ECG |
| HRV - High Frequency Power -slope 4 | 4 day slope of named feature | ECG |
| HRV - LF_HF Ratio | Low frequency/ High frequency ratio from ECG | ECG |
| HRV - LF_HF Ratio - normalized | Normalized version of named feature | ECG |
| HRV - LF_HF Ratio - slope4 | 4 day slope of named feature | ECG |
| HRV - ln_RMSSD | Natural logarithm of Root Mean Square of Successive Differences between normal heartbeats measured in milliseconds | ECG |
| HRV - ln_RMSSD - normalized | Normalized version of named feature | ECG |
| HRV - ln_RMSSD - slope4 | 4 day slope of named feature | ECG |
| HRV - Low Frequency Power | Low frequency | ECG |
| HRV - Low Frequency Power - normalized | Normalized version of named feature | ECG |
| HRV - Low Frequency Power - slope4 | 4 day slope of named feature | ECG |
| HRV - pNN50 | Percentage of absolute differences in successive NN values > 50 ms | ECG |
| HRV - pNN50 - normalized | Normalized version of named feature | ECG |
| HRV - pNN50 - slope4 | 4 day slope of named feature | ECG |
| HRV - RMSSD | Root Mean Square of Successive Differences between normal heartbeats measured in milliseconds | ECG |
| HRV - RMSSD - normalized | Normalized version of named feature | ECG |
| HRV - RMSSD - slope4 | 4 day slope of named feature | ECG |
| HRV - SDNN | Standard Deviation of Normal-to-Normal interbeat intervals measured in milliseconds | ECG |
| HRV - SDNN - normalized | Normalized version of named feature | ECG |
| HRV - SDNN - slope4 | 4 day slope of named feature | ECG |
| HRV - SDNNI | Mean of standard deviations of all NN intervals for each 5 min segment of 24-h HRV recording | ECG |
| HRV - SDNNI - normalized | Normalized version of named feature | ECG |
| HRV - SDNNI - slope 4 | 4 day slope of named feature | ECG |
| HRV - SDRR | Standard deviation in time between heartbeats | ECG |
| HRV - SDRR - normalized | Normalized version of named feature | ECG |
| HRV - SDRR - slope4 | 4 day slope of named feature | ECG |
| HRV - SI | Stress Index | ECG |
| HRV - SI - slope4 | 4 day slope of named feature | ECG |
| HRV - SI -normalized | Normalized version of named feature | ECG |
| RR - Bradypnoea (%) | Percentage of time where RR is below 12 | ECG |
| RR - Bradypnoea (%) - slope4 | 4 day slope of named feature | ECG |
| RR - I:E Ratio | Inspiration/expiration ratio | ECG |
| RR - I:E Ratio - max - normalized | Normalized version of named feature | ECG |
| RR - I:E Ratio - mode - normalized | Normalized version of named feature | ECG |
| RR - I:E Ratio - normalized | Normalized version of named feature | ECG |
| RR - I:E Ratio - slope4 | 4 day slope of named feature | ECG |
| RR - max | Maximum respiration rate | ECG |
| RR - max - normalized | Normalized version of named feature | ECG |
| RR - max - slope4 | 4 day slope of named feature | ECG |
| RR - mean | mean respiration rate from heartpy algorithm | ECG |
| RR - min | Minimum respiration rate | ECG |
| RR - min - normalized | Normalized version of named feature | ECG |
| RR - min - slope4 | 4 day slope of named feature | ECG |
| RR - Tachypnoea (%) | Percentage of time where RR is above 25 | ECG |
| RR - Tachypnoea (%) - slope4 | 4 day slope of named feature | ECG |
| Temperature - difference to mean - area | Area between mean temperature for a subject, and current day temperature curves | Temperature |
| Temperature - difference to mean - mae | Mean absolute error between mean temperature and current day temperature curves | Temperature |
| Temperature - difference to mean - mse | Mean standard error between mean temperature and current day temperature curves Temperature | Temperature |
| Temperature - difference to mean - pcm | Partial curve mapping between mean temperature and current day temperature curves | Temperature |
| Temperature - max | Maximum temperature | Temperature |
| Temperature - max - normalized | Normalized version of named feature | Temperature |
| Temperature - max - slope4 | 4 day slope of named feature | Temperature |
| Temperature - mean | Average temperature | Temperature |
| Temperature - mean - normalized | Normalized version of named feature | Temperature |
| Temperature - mean - slope4 | 4 day slope of named feature | Temperature |
| Temperature - min | Minimum temperature | Temperature |
| Temperature - min - normalized | Normalized version of named feature | Temperature |
| Temperature - min - slope4 | 4 day slope of named feature | Temperature |

Standard analyses and descriptive statistics were carried out using JMP 18.0 (SAS Institute Inc., Cary, NC, USA) or higher. Machine learning models were implemented using Python 3.10 or higher (The Python Software Foundation, Wilmington, Delaware, USA). The following main packages for statistical learning were used in the indicated version or higher: Catboost 1.2.7, keras 2.10.0, LightGBM 4.6.0, mlforecast 1.0.2, neuralforecast 1.7.7, numba 0.57.1, numpy 1.24.4, optuna 4.2.1, pandas 2.0.3, polars 1.26.0, pytorch 2.5.1, scikit-learn 1.6.1, scipy 1.11.3, shap 0.46, statsmodels 0.14.0, tensorflow 2.10.0, XGBoost 2.1.3. Imputations were made using Last Observation Carried Forward (LOCF) where necessary. Training and testing sets were generated in a time-sequential order by either taking a fixed number of days or fixed percentage of days per subject for training, or by taking all data from all subjects excluding one and adding only a fixed number of days from the selected subject. Hyperparameter optimization was done using optuna. The patient identifier (original_id) was modeled as categorical (in tensorflow as feature_column.categorical_column_with_hash_bucket), the other features as continuous. Continuous features were normalized for LSTM and CNN approaches.

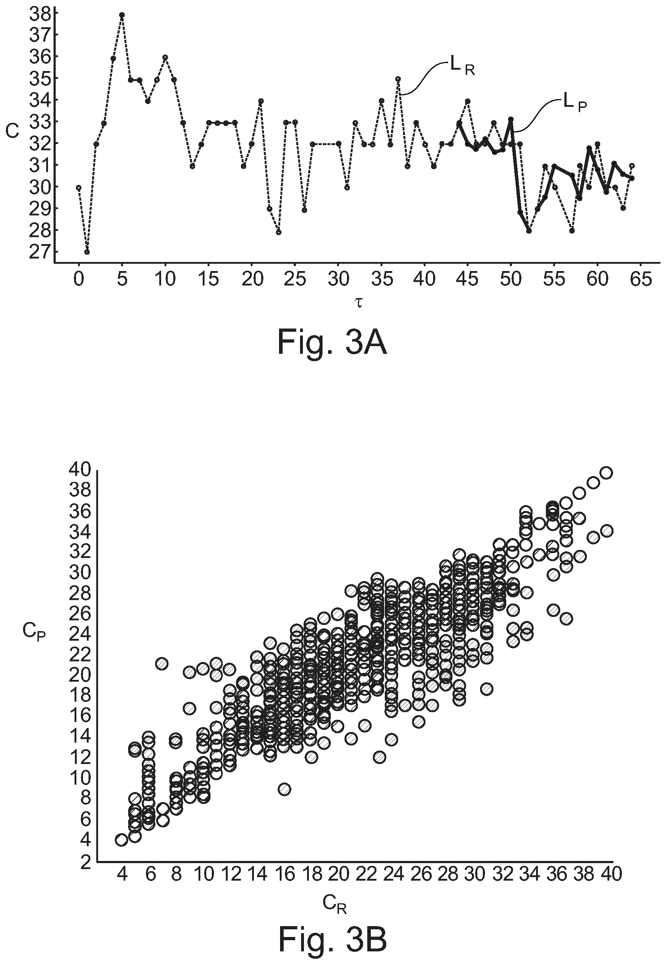

Fig. 3A-B schematically depict results obtained using a trained model that was trained using the XGBoost training algorithm and 90 data features to predict the CAT-score of a target subject 55 six days in advance, i.e., with a target date 6 days after the last date.

Fig. 3A schematically depicts subject-reported CAT-scores 45 and CAT-score predictions 43 over time τ (in days) for a single target subject 55. Specifically, line L_{R} corresponds to the subject-reported CAT-scores 45 and line L_{P} to the CAT-score predictions. As may be observed in Fig. 3A, the trained model 33 successfully predicted the trend in the CAT-score of the target subject 55 several days in advance. Hence, the prediction method of the invention may facilitate providing an accurate patient-reported outcome prediction six days in advance based on physiological data gathered using a sensor device worn by the patient.

Fig. 3B schematically depicts CAT-scores predictions C_{P} on the y-axis versus subject-reported CAT-scores C_{P} on the x-axis for the subject population. As may be observed in Fig. 3B, the CAT-score predictions (six days in advance) correlated well with the subject-reported CAT-scores (R² = 0.826).

Above-mentioned trained model 33 was further checked for which parts of the input data 5 contribute most to accurate CAT-score predictions. All input data 5 were provided as single values per day. The top contributions were observed for the prior CAT-scores 40 (even when only using second CAT-score predictions 84), a respiration rate feature corresponding to a percentage of time with a breathing rate > 25/min; the heart rate feature corresponding to a percentage of time where HR<50; a heart rate variability feature corresponding to the standard deviation in time between heartbeats; and an activity feature corresponding to an accelerometer cluster. The specific top-performing data features may vary between different trained models 33, e.g., based on the division of training data and testing data, based on the training algorithm, or due to an inherent variation in model training procedures, however, the top-performing features consistently contained the subject identifier, the prior CAT-scores, at least one respiration rate feature, at least one heart rate feature, at least one heart rate variability feature, and at least one activity feature. Hence, in embodiments, the data features may comprise (at least) a respiration rate feature, a heart rate feature, a heart rate variability feature, and an activity feature.

Other typically top-performing feature categories include temperature features and coughing features. For instance, for above-mentioned trained model 33 (also see Fig. 3A and 3B), the top-performing features included the temperature features (i) normalized minimal temperature (15 min intervals), (ii) maximal temperature (15 min intervals), (iii) average temperature; and a cough feature corresponding to the coughing frequency. Hence, in further embodiments, the data features may include a temperature feature and/or a coughing feature, especially at least a temperature feature, or especially at least a coughing feature.

The trained model 33 described above was obtained by training a model 30 using the XGBoost algorithm with a 50-50 split of available data as training data and as testing data. Similar - yet improved - results were obtained using a 70-30 split with XGBoost as well as with other training algorithms, such as with a Long Short Term Memory (LSTM) training algorithm. Accordingly, in embodiments, the supervised training algorithm may be selected from the group comprising a gradient boosting algorithm such as XGBoost, lightGBM, Catboost, a random forest algorithm, a neural network algorithm, such as a NN capable of handling time-series data such as LSTM, 1D CNN, GRU, RNN, TCN, TimesNet, BiTCN, DeepAR, NBEATS, NBEATSx, NHITS, TiDE, DeepNPTS, TSMixer, TSMixerx, MLPMultivariate, DLinear, NLinear, TFT, Informer, Autoformer, FedFormer, PatchTST, iTransformer, StemGNN, and Time-LLM. In further, the supervised training algorithm may be selected from the group consisting of XGBoost, lightGBM, LSTM, 1D CNN, GRU, NHITS, Informer, Autoformer, and Time-LLM, especially from the group consisting of XGBoost and LSTM.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiment" and similar terms may refer to an embodiment comprising the features of the previously discussed embodiment but may also refer to an alternative embodiment.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. **In** a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. **A computer-implemented** method for providing a CAT-score prediction (43) for a target subject (55) and a target date using a trained model (33), wherein the computer-implemented method comprises:
- generating data features (15) from physiological data (10) on the target subject (55), wherein the physiological data (10) comprises electrocardiogram data (11), temperature data (12), and motion data (13), and wherein the data features (15) comprise a respiration rate feature, a heart rate variability feature, a heart rate feature, and an activity feature; and
- providing input data (5) to the trained model (33) and generating, by the trained model (33), the CAT-score prediction (43) based on the input data (5), wherein the input data (5) comprises (i) the data features (15), (ii) prior CAT-scores (40), and (iii) target subject information (20) for the target subject (55), wherein the prior CAT-scores (40) precede the target date, wherein the target subject information (20) comprises a subject identifier for the target subject (55).

2. The computer-implemented method according to claim 1, wherein the input data (5) comprises a plurality of timepoints, wherein for each timepoint the input data (5) includes values for the data features (15) and for the prior CAT-scores (40).

3. The computer-implemented method according to any one of the preceding claims, wherein the physiological data (10) and the prior CAT-scores (40) correspond to a timeframe of n days, wherein n ≥ 3.

4. The computer-implemented method according to claim 3, wherein the timeframe includes a last date, wherein the target date is selected from the range of 1 - 14 days after the last date.

5. The computer-implemented method according to claim 4, wherein the computer-implemented method comprises:
- generating second data features (85) from the physiological data, wherein the second data features (85) comprise a respiration rate feature, a heart rate variability feature, a heart rate feature, and an activity feature; and
- providing second input data to a second trained model (83), and generating, by the second trained model (83), at least part of the prior CAT-scores, wherein the second input data (86) comprises the second data features and the target subject information (20), wherein the second trained model (83) is configured to provide second CAT-score predictions (84) based on the second input data (86).

6. The computer-implemented method according to any one of the preceding claims, wherein the trained model (33) is trained to provide CAT-score predictions (43) using training data (60), wherein the training data (60) includes training data features (65), training prior CAT-scores (64) for a subject population (50) with subject population information (62), wherein the subject population (50) includes the target subject (55) covering a target subject training timeframe of at least 14 days.

7. The computer-implemented method according to any one of the preceding claims, wherein:
- the respiration rate feature is selected from the group comprising an atypical breathing episode feature and a mean respiration rate;
- the heart rate variability feature is selected from the group comprising a standard deviation of an interbeat interval, a stress index, and a proportion of successive Normal-to-Normal intervals exceeding a predefined duration;
- the heart rate feature is selected from the group comprising a mean heart rate, a maximum heart rate, and a tachycardia episode feature; and
- the activity feature is selected from the group comprising a motion data cluster, an activity frequency, and a step count.

8. The computer-implemented method according to any one of the preceding claims, wherein the data features further comprise a temperature feature, wherein the temperature feature is selected from the group comprising a minimum temperature and a maximum temperature, and wherein the subject information (20) further comprises baseline features and demographic data.

9. The computer-implemented method according to any one of the preceding claims, wherein the data features further comprise a cough feature, wherein the cough feature is selected from the group comprising a cough frequency and a cough frequency change, and wherein the cough feature is generated based on one or more of the electrocardiogram data (11) and the motion data (13).

10. The computer-implemented method according to any one of the preceding claims, wherein the computer-implemented method further comprises:
- acquiring the physiological data (10) on the subject (55) from a sensor arrangement (130).

11. The computer-implemented method according to any one of the preceding claims, wherein the computer-implemented method further comprises:
- periodically requesting the target subject (55) to provide user input to determine subject-reported CAT-scores;
- comparing subject-reported CAT-scores to CAT-score predictions to determine a prediction performance; and
- when the prediction performance falls below a predefined performance threshold:
- re-training the trained model using the subject-reported CAT-scores and corresponding physiological data; or
- transmitting a performance notification to indicate that the prediction performance for the target subject (55) has fallen below the predefined performance threshold.

12. The computer-implemented method according to any one of the preceding claims, wherein the computer-implemented method further comprises:
- comparing the CAT-score prediction to a predefined alert threshold, and, if the CAT-score prediction exceeds the predefined alert threshold, transmitting an exceedance notification to indicate that the CAT-score has exceeded the predefined alert threshold.

13. A computer-implemented training method for providing a trained model (33), wherein the trained model (33) is configured for providing a CAT-score prediction (43) for a target subject (55), wherein the computer-implemented training method comprises:
- generating training data (60) for a subject population (50) of subjects (56) based on physiological data (10) on the subjects (56), wherein the subject population (50) includes the target subject (55), wherein the physiological data (10) comprises electrocardiogram data (11), temperature data (12), and motion data (13) covering a timeframe of at least 14 days for the target subject (55); wherein for each subject (56) in the subject population (50) the training data (60) comprises data features (15), prior CAT-scores (40), subject information (20), and target CAT-scores, wherein:
- the data features (15) comprise a respiration rate feature, a heart rate variability feature, a heart rate feature, and an activity feature;
- the subject information (20) comprises subject identifiers;
- training a model (30) based on the training data (60) using a supervised training algorithm in order to provide model parameters (36); and
- providing a trained model (33) based on the model (30) and the model parameters (36).

14. The computer-implemented training method according to claim 13, wherein the subject population (50) the target subject (55) and ≥ 30 other subjects (56), wherein the training data (60) covers a timeframe of at least 30 days for the other subjects (56), and wherein for each subject (56) applies that:
- the training data (60) comprises a plurality of timepoints, wherein the data features (15) and the prior CAT-scores (40) include values for each of the timepoints.

15. The computer-implemented training method according to any one of the preceding claims 13-14, wherein:
- the respiration rate feature is selected from the group comprising an atypical breathing episode feature and a mean respiration rate;
- the heart rate variability feature is selected from the group comprising a standard deviation of an interbeat interval, a stress index, and a proportion of successive Normal-to-Normal intervals exceeding a predefined duration; and
- the heart rate feature is selected from the group comprising a mean heart rate, a maximum heart rate, and a tachycardia episode feature.

16. The computer-implemented training method according to any one of the preceding claims 13-15, wherein the supervised training algorithm is selected from the group consisting of XGBoost, Catboost, lightGBM, LSTM, 1D CNN, GRU, NHITS, Informer, Autoformer, and Time-LLM.

17. The computer-implemented training method according to any one of the preceding claims 13-16, wherein the computer-implemented training method further comprises:
- acquiring physiological data (10) on the subject population (50), wherein the physiological data (10) comprises electrocardiogram data (11), temperature data (12), and motion data (13).

18. A data processing system (300) comprising means for carrying out the steps of the computer-implemented method according to any one of the preceding claims 1-12 and/or of the computer-implemented training method according to any one of the preceding claims 13-17.

19. A system (100) for providing a CAT-score prediction (43), wherein the system (100) comprises the data processing system (300) of claim 18 and a sensor arrangement (130), wherein the sensor arrangement (130) comprises an electrocardiogram sensor (131), a temperature sensor (132), and a motion sensor (133), wherein the sensor arrangement (130) is configured to determine physiological data (10) of a target subject (55) and to provide the physiological data to the data processing system (300), wherein the physiological data (10) comprises electrocardiogram data (11), temperature data (12), and motion data (13).

20. The system (100) according to claim 19, wherein the system (100) comprises a wearable device (110), wherein the wearable device (110) comprises the sensor arrangement (130), wherein the sensor arrangement (130) is configured to determine the physiological data (10) of the target subject (55) when the target subject (55) is wearing the wearable device (110).

21. A computer program product comprising instructions for execution on a data processing system (300), wherein the instructions, when executed by the data processing system (300), cause the data processing system (300) to carry out the computer-implemented method according to any one of the preceding claims 1-12 and/or the computer-implemented training method according to any one of the preceding claims 13-17.

22. A data carrier (310), carrying thereupon program instructions which, when executed by a data processing system (300), cause the data processing system (300) to carry out the computer-implemented method according to any one of the preceding claims 1-12 and/or the computer-implemented training method according to any one of the preceding claims 13-17.

23. A trained model (33) obtainable by the computer-implemented training method according to any one of the preceding claims 13-17, wherein the trained model (33) is configured to:
- receive input data (5) on a target subject (55), wherein the input data (5) comprises data features (15), prior CAT-scores (40), and subject information (20), wherein the data features (15) comprise a respiration rate feature, a heart rate variability feature, a heart rate feature, and an activity feature, wherein the prior CAT-scores (40) precede a target date, and wherein the subject information (20) comprises a subject identifier; and
- provide a CAT-score prediction (43) for the target subject (55) on the target date based on the input data (5).

24. A data carrier (310) carrying thereupon the trained model according to claim 23.
